# DEMANDE DE BREVET EUROPEEN

(11) **EP 0 783 876 A1**
(43) Date de publication de la demande: **16.07.1997**
(21) Numéro de dépôt: 96420007.5
(22) Date de dépôt: 09.01.1996
(51) Int. Cl.: A61F 13/04

(54) **Semelle d'appui pour plâtre de marche**

(71) Demandeur: Rousseau, Bernard, Noumea (NC); Bracq, Christian, Noumea (NC)
(72) Inventeur: Rousseau, Bernard, Noumea, Nouvelle Caledonie (FR)
(74) Mandataire: Laurent, Michel

(57) **Abrégé**

Semelle d'appui (1) pour plâtre de marche, comportant essentiellement un corps (ou embase) (2) dont la surface supérieure (3) sert d'appui à la plante du pied par l'intermédiaire d'un soutien de voûte plantaire (4), et en dessous duquel sont disposés trois blocs d'appui transversaux (5,6,7), un à chaque extrémité et le troisième dans la partie médiane, les bandes formant le bandage pour réaliser le plâtre passant de part et d'autre desdites zones d'appui (5,6,7), et dans laquelle l'embase (2), le soutien de voûte plantaire (4) et les appuis (5,6,7) associés à ladite embase, sont disposés de part et d'autre d'un plan de symétrie transversal XX permettant une utilisation aussi bien à droite qu'à gauche.

Elle se caractérise le soutien de voûte plantaire (4) s'étend sur environ la moitié de la largeur de l'embase (2) et déborde latéralement, la zone d'appui intermédiaire (7), disposée sous la face inférieure, s'étendant, quant à elle, uniquement sur environ la moitié de la largeur de ladite embase (2), et ce du côté opposé à l'axe longitudinal YY de cette dernière par rapport au soutien de voûte plantaire (4).

## Description

La présente invention a trait à une semelle d'appui pour plâtre de marche permettant d'obtenir non seulement une meilleure stabilité lors de la marche, mais également un confort amélioré pour l'utilisateur. Dans la suite de la description, le terme "plâtre" sera utilisé dans un sens très général pour désigner non seulement les plâtres conventionnels, mais également ceux réalisés à partir d'une résine.

Chez les blessés du membre inférieur dont l'état des lésions permet un appui avec immobilisation par plâtre, et pour lesquels la marche est autorisée, la pression sur le sol ne se fait pas directement de la substance plâtrée ou de la résine sur le sol, mais toujours par l'intermédiaire d'un élément incorporé à l'intérieur du plâtre.

La solution la plus utilisée encore de nos jours, consiste à incorporer dans le plâtre une talonnette. Ces talonnettes sont de forme cylindrique et ont un appui plane à la surface supérieure, et ne soutiennent donc pas la voûte plantaire.

Par ailleurs, leur surface inférieure de section cylindrique entraîne une instabilité dans l'appui, pouvant entraîner des douleurs dans les genoux.

Leurs avantages qui font qu'elles sont encore couramment utilisées, résident dans le fait qu'elles sont économiques à fabriquer, qu'elles peuvent indifféremment se placer sous un pied droit ou un pied gauche et, qu'en général, deux tailles suffisent pour équiper des individus pouvant avoir des morphologies très différentes (enfants, adultes).

Pour surmonter les inconvénients que présentent de telles talonnettes, il a été cependant proposé depuis fort longtemps de les remplacer par des éléments de type "semelle", comme cela ressort notamment des brevets français 1 595 180, 2 504 386 et du EP-A-0 044 459.

La solution décrite dans le premier de ces documents est complexe, difficile à mettre en oeuvre et surtout implique d'avoir des éléments adaptés à chaque taille de pied et différents en ce qui concerne le pied droit et le pied gauche.

Celle faisant l'objet du brevet 2 504 386, est séduisante, mais implique également d'avoir un type de semelle spécifique pour chaque taille de pied, et nécessite également un modèle pour le pied droit et un pour le pied gauche.

Enfin, si la solution de l'EP-A-0 044 459 permet d'envisager une utilisation aussi bien pour un pied droit qu'un pied gauche, elle est complexe, n'assure pas une très bonne stabilité et manque de confort par le fait qu'elle ne permet pas de reproduire au plus près l'appui normal d'un pied.

Or on a trouvé, et c'est ce qui fait l'objet de la présente invention, un nouveau type de semelle facilement incorporable lors de la réalisation d'un plâtre de marche qui non seulement peut être adapté indifféremment sous le pied droit ou le pied gauche, mais qui en outre, assure une stabilité et un confort amélioré par rapport aux solutions antérieurement proposées.

D'une manière générale, l'invention concerne donc un nouveau type de semelle d'appui pour plâtre de marche, qui comporte essentiellement un corps (ou embase) en un matériau semi-rigide dont la surface supérieure sert d'appui à la plante du pied par l'intermédiaire d'un soutien de voûte plantaire, et en dessous duquel sont disposés trois blocs d'appui transversaux, un à chaque extrémité et le troisième dans la partie médiane, les bandes formant le bandage pour réaliser le plâtre passant de part et d'autre desdites zones d'appui, et dans laquelle l'embase, le soutien de voûte plantaire et les appuis associés à ladite embase, sont disposés de part et d'autre d'un plan de symétrie transversal permettant une utilisation aussi bien à droite qu'à gauche.

La semelle selon l'invention se caractérise en ce que le soutien de voûte plantaire s'étend sur environ la moitié de la largeur de ladite embase et déborde latéralement, la zone d'appui intermédiaire, disposée sous la face inférieure, s'étendant, quant à elle, uniquement sur environ la moitié de la largeur de ladite embase, et ce du côté opposé à l'axe longitudinal de cette dernière par rapport à la voûte plantaire.

Selon une forme de réalisation, l'embase est de forme rectangulaire et comporte, à ses deux extrémités, selon l'axe longitudinal, deux ergots autour desquels passe également le bandage lors de la réalisation du plâtre.

Conformément à l'invention, l'appui antérieur se trouve quant à lui à peu près à la hauteur de la tête des métatarsiens et l'appui postérieur se trouve à peu près à la hauteur de la surface d'appui du talon.

Grâce à une telle forme de réalisation, lorsque la semelle ainsi réalisée est fixée, par l'intermédiaire des bandes plâtréees ou des bandes de résine sous le pied, elle permet de reproduire au plus près l'appui normal d'un pied qui se fait au niveau des surfaces des têtes des métatarsiens, au niveau du talon, du calcanéum et également au niveau du cinquième métatarsien.

En conséquence, les forces d'appui du pied se situent donc en avant, en arrière, mais également latéralement, la voûte plantaire repoussant en dehors les forces médianes grâce à l'appui médian qui ne s'étend que sur environ la moitié de la largeur de ladite semelle.

Une telle disposition des points d'appui permet au mieux de se rapprocher des appuis physilogiques et facilite également la fixation de la semelle au plâtre en faisant passer les bandes plâtrées ou les bandes de résine en X entre les points d'appui.

Dans une forme préférentielle de réalisation conforme à l'invention, les surfaces inférieures des blocs d'appui s'inscrivent, vu de profil, dans un arc de cercle. Une telle forme de réalisation permet d'avoir un déroulement minime du pied à la marche et, par ailleurs, permet de compenser une légère erreur dans la construction de plâtre, par exemple une angulation de 100 degrés au lieu de 90° ; dans cette forme de réalisation préférentielle, des renforcements sont également prévus, dans la semelle, entre les blocs d'appui.

Par ailleurs, les blocs d'appui inférieurs peuvent éventuellement présenter des dessins anti-dérapants.

Une telle semelle est réalisée dans un matériau semi-rigide et peut être faite soit d'un ensemble monobloc, par exemple par moulage de caoutchouc, soit en associant entre elles, par exemple par collage, des pièces élémentaires, l'une en caoutchouc plane correspondant à la semelle, et la dernière, permettant de réaliser le soutien de la voûte plantaire, dans un matériau plus souple tel que par exemple en liège.

D'un point de vue fabrication, il est cependant avantageux de réaliser une telle semelle par moulage en un seul bloc.

L'invention et les avantages qu'elle apporte seront cependant mieux compris grâce à l'exemple de réalisation donné ci-après à titre indicatif mais non limitatif, et qui est illustré par les figures annexées dans lesquelles :
- la figure 1 est une vue schématique en perspective éclatée d'une semelle réalisée conformément à l'invention ;
- les figures 2,3 et 4 sont respectivement des vues de dessus, de côté et en coupe transversale selon l'axe XX de la figure 1 d'une semelle conforme à l'invention.

Si l'on se reporte aux schémas annexés, la semelle d'appui pour plâtre de marche conforme à l'invention, désignée par la référence générale (1), se compose donc essentiellement d'une embase (2) en un matériau semi-rigide dont la surface supérieure (3) sert d'appui à la plante du pied et comporte un soutien de voûte plantaire (4). En dessous de cette embase (3), sont disposés trois blocs d'appui transversaux (5,6,7). Par ailleurs, deux ergots (8,9) sont disposés à chaque extrémité de l'embase (3), et ce selon l'axe longitudinal de l'embase (3).

Ainsi que cela ressort des schémas annexés, conformément à l'invention, l'embase (3) comporte deux axes de symétrie XX-YY et le soutien de voûte plantaire (4) ainsi que les appuis (5,6,7) sont disposés de part et d'autre du plan de symétrie transversal XX, ce qui permet une utilisation d'une telle semelle aussi bien pour équiper un pied droit qu'un pied gauche.

Par ailleurs, le soutien de voûte plantaire (4) dont la structure ressort clairement des dessins annexés a une forme telle qu'il constitue un relief commençant au milieu (20,21) des points d'appui postérieur et antérieur (5,6) et culmine à un niveau (22), aux milieux de la longueur, le sommet (22) étant déjeté vers l'extérieur du bord (10). La voûte plantaire se termine en pente douce au niveau de la prolongation de la projection de l'extrémité interne de l'appui médian inférieur (7) au milieu de cette projection. D'une manière générale, on peut dire que le soutien de voûte plantaire (4) s'étend donc sur environ la moitié de la largeur de l'embase (2), et déborde latéralement par rapport au côté (10) de cette dernière.

La zone d'appui intermédiaire (7) disposée sur la face inférieure (11) de l'embase (2), s'étend également uniquement sur environ la moitié de la largeur de l'embase, et ce du côté opposé à l'axe longitudinal YY de cette dernière par rapport à la voûte plantaire (4).

Dans le mode de réalisation illustré, l'appui intermédiaire (7) a une section de forme générale rectangulaire qui décroît progressivement de l'extérieur vers l'extrémité située au voisinage de l'axe YY.

Les deux blocs extrêmes (5,6) s'étendent, quant à eux, sur toute la largeur de l'embase.

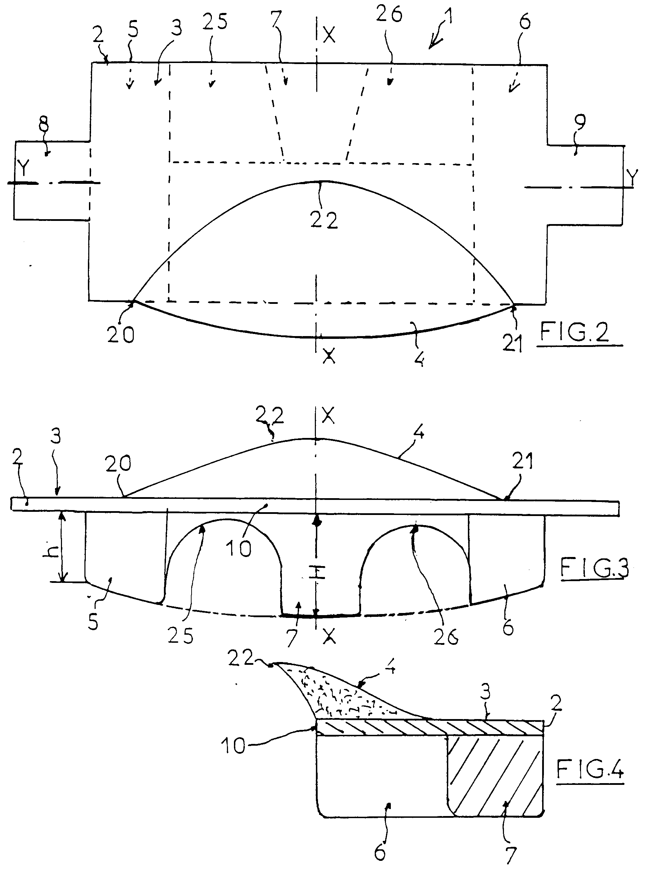

Par ailleurs, ainsi que cela ressort plus clairement de la figure 3, les surfaces d'appui (5,6,7) s'inscrivent dans un arc de cercle. Une telle configuration permet ainsi, d'une part, un déroulement minime du pied à la marche et d'autre part, de compenser une éventuelle légère erreur dans la construction du plâtre lorsque le pied n'est pas posé exactement à 90°, mais par exemple avec une angulation de l'ordre de 100°.

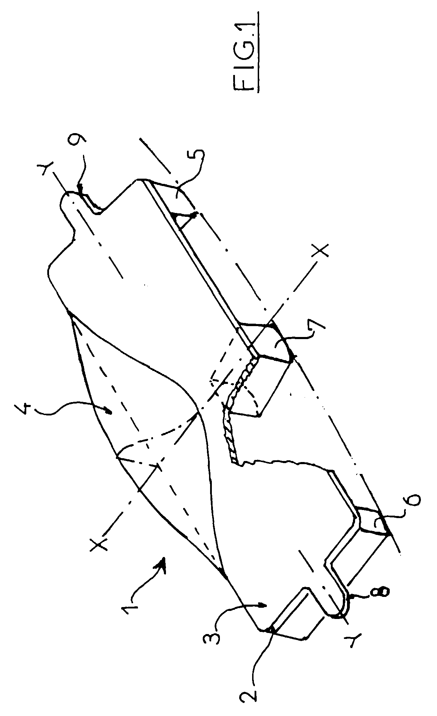

Enfin, la zone située en dessous de l'embase (2) comprise entre les blocs extrêmes (5,6) et le bloc intermédiaire (7), comporte des renforcements (25,26) avantageusement en forme d'arche (éléments non représentés à la figure 1).

Une semelle ainsi réalisée peut, comme dit précédemment, être utilisée aussi bien pour équiper un pied droit qu'un pied gauche.

Par ailleurs, il peut être envisagé d'équiper la plupart des patients en ayant seulement deux tailles différentes.

A titre indicatif, les caractéristiques de deux semelles, l'une de petite taille référencée A, l'autre de grande taille référencée B, seront les suivantes.

### Embase (2) :

Longueur : A = 16 cm ; B = 18 cm
Largeur : A = 6 cm ; B = 7,5 cm
Epaisseur : A-B : 4 mm

### Ergots (8,9) :

Longueur : A = 2 cm ; B = 2 cm
Largeur : A = 2 cm ; B = 2,5 cm

### Blocs d'appuis (5,6,7) :

En se reportant à la figure 3, la surface inférieure des appuis s'inscrit dans un arc de cercle de rayon 18,9 cm pour une petite taille A et 25 cm pour un modèle de grande taille B, la hauteur h aux extrémités étant de 1,8 cm pour le modèle A et 2 cm pour le modèle B, et la hauteur H dans l'axe XX étant respectivement de 2,7 cm (modèle A) et 3 cm (modèle B).

Les blocs d'appui (5,6) s'étend sur toute la largeur de la semelle, alors que le bloc d'appui (7) s'étend sur environ la moitié de la largeur de l'embase (2), à savoir sur une longueur de 2,5 cm pour le modèle A et une longueur de 3,5 cm pour le modèle B.

La section de l'appui médian (7) est sensiblement rectangulaire, cet appui ayant avantageusement une largeur décroissante en allant de l'extérieur oil elle est de 2,5 cm pour un modèle A et 3 cm pour un modèle B et vers le bord interne où elle est de 1,5 cm pour un modèle A et 2 cm pour un modèle B.

### Soutien de voûte plantaire (4) :

Hauteur du point le plus haut (22) par rapport à l'embase (3) :
A = 1,5 cm ; B = 2 cm. Déport par rapport au côté (10) de l'embase (3) : A-B = 1 cm.

Grâce à une telle structure, lorsque la semelle est incorporée au plâtre, on obtient une reproduction pratiquement parfaite d'un appui normal d'un pied, l'appui se faisant au niveau des surfaces des têtes des métatarsiens, au niveau du talon, au niveau du calcanéum et également au niveau du cinquième métatarsien.

Par ailleurs, les forces d'appui du pied s'exerçant non seulement en avant et en arrière, mais également latéralement, la voûte plantaire repoussant vers l'extérieur les forces médianes grâce au positionnement et dimensionnement de l'appui médian.

Enfin, lorsque le plâtre n'est pas parfaitement à 90° de flexion du pied sur jambe, et qui se trouve donc légèrement en équin, on peut compenser cet équin en passant les bandes permettant de réaliser le plâtre sous l'appui postérieur.

Bien entendu, l'invention n'est pas limitée aux exemples de réalisation décrits précédemment, mais elle en couvre toutes les variantes réalisées dans le même esprit.

## Revendications

1. Semelle d'appui (1) pour plâtre de marche, comportant essentiellement un corps (ou embase) (2) dont la surface supérieure (3) sert d'appui à la plante du pied par l'intermédiaire d'un soutien de voûte plantaire (4), et en dessous duquel sont disposés trois blocs d'appui transversaux (5,6,7), un à chaque extrémité et le troisième dans la partie médiane, les bandes formant le bandage pour réaliser le plâtre passant de part et d'autre desdites zones d'appui (5,6,7), et dans laquelle l'embase (2), le soutien de voûte plantaire (4) et les appuis (5,6,7) associés à ladite embase, sont disposés de part et d'autre d'un plan de symétrie transversal XX permettant une utilisation aussi bien à droite qu'à gauche, caractérisée le soutien de voûte plantaire (4) s'étend sur environ la moitié de la largeur de l'embase (2) et déborde latéralement, la zone d'appui intermédiaire (7), disposée sous la face inférieure, s'étendant, quant à elle, uniquement sur environ la moitié de la largeur de ladite embase (2), et ce du côté opposé à l'axe longitudinal YY de cette dernière par rapport au soutien de voûte plantaire (4).

2. Semelle selon la revendication 1, caractérisée en ce que les surfaces inférieures des appuis (5,6,7) s'inscrivent dans un arc de cercle, des zones de renforcement étant prévues entre lesdits appuis en dessous de l'embase (2).

3. Semelle selon l'une des revendications 1 et 2, caractérisée en ce que l'embase (2) est de forme rectangulaire et comporte, à ses deux extrémités, selon l'axe longitudinal YY, deux ergots (8,9) autour desquels passe également le bandage lors de la réalisation du plâtre.

4. Semelle selon l'une des revendications 1 et 3, caractérisée en ce que les blocs d'appui inférieurs (5,6,7) présentent des dessins antidérapants.

5. Semelle selon l'une des revendications 1 à 4, caractérisée en ce qu'elle est réalisée en une seule pièce par moulage.

6. Semelle selon l'une des revendications 1 à 4, caractérisée en ce qu'elle est réalisée par assemblage de pièces élémentaires, par exemple par collage.
